(19) Europäisches Patentamt
European Patent Office
Office européen des brevets

(11) **EP 3 204 437 B1**

(12) # EUROPEAN PATENT SPECIFICATION

(45) Date of publication and mention
of the grant of the patent:
**17.07.2024 Bulletin 2024/29**

(21) Application number: **15848841.1**

(22) Date of filing: **06.10.2015**

(51) International Patent Classification (IPC):
**C08L 53/02** *(2006.01)* **C08L 23/20** *(2006.01)*
**C08L 9/06** *(2006.01)* **A61L 15/58** *(2006.01)*
**B32B 27/32** *(2006.01)*

(52) Cooperative Patent Classification (CPC):
(C-Sets available)
**A61L 15/585; B32B 5/02; B32B 7/12; B32B 27/12;
B32B 27/32; C08L 23/20; C08L 53/025;**
B32B 2250/03; B32B 2250/40; B32B 2262/0215;
B32B 2262/0292; B32B 2307/51; B32B 2307/548;
B32B 2555/02; C08L 2205/025; (Cont.)

(86) International application number:
**PCT/US2015/054131**

(87) International publication number:
**WO 2016/057452 (14.04.2016 Gazette 2016/15)**

(54) **ADHESIVE COMPOSITIONS WITH AMORPHOUS POLYOLEFINS**

KLEBSTOFFZUSAMMENSETZUNGEN MIT AMORPHEN POLYOLEFINEN

COMPOSITIONS ADHÉSIVES AVEC DES POLYOLÉFINES AMORPHES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.10.2014 US 201414510528**

(43) Date of publication of application:
**16.08.2017 Bulletin 2017/33**

(73) Proprietor: **Kraton Polymers U.S. LLC
Houston, Texas 77084 (US)**

(72) Inventor: **DUBOIS, Donn
Houston, Texas 77079 (US)**

(74) Representative: **Henkel & Partner mbB
Patentanwaltskanzlei, Rechtsanwaltskanzlei
Maximiliansplatz 21
80333 München (DE)**

(56) References cited:
**EP-A1- 0 857 758** **WO-A1-98/50465**
**WO-A2-03/044087** **US-A1- 2010 305 528**
**US-A1- 2011 244 232** **US-A1- 2013 225 020**
**US-A1- 2014 272 214**

**EP 3 204 437 B1**

**(Cont. next page)**

(52) Cooperative Patent Classification (CPC): (Cont.)
C08L 2205/035

C-Sets
**A61L 15/585, C08L 53/02;**
**C08L 53/025, C08L 23/02, C08L 91/00,**
**C08L 53/025**

**Description**

**Field of the Invention**

The invention relates to a composition and preferably hot melt adhesive composition that, when used as an elastic attachment adhesive (EAA) to construct elastic laminates, provides an initial creep performance of less than 25%. The composition is typically employed in elastic attachment adhesives (EAA) used for laminating elastic fibers in diaper leg dam construction or adult incontinent articles. These adhesives attach a series of stretched elastic fibers, typically thermoplastic polyurethane (TPU), to a laminate of polyolefin-based films. The composition comprises low viscosity, high vinyl, styrene - ethylene/butadiene - styrene (SEBS) block copolymers, amorphous polyolefins such as polyethylene, polypropylene, butylene homopolymers and copolymers, or a mixture of two or more of these, tackifier, and maleated polypropylene oligomer or maleated SEBS.

**Background of the Invention**

Generally styrene-isoprene-styrene (SIS) block copolymers with about 30% polystyrene content (PSC) are formulated with hydrocarbon resin(s) (HCR), polystyrene (PS) endblock resin, and a small amount of plasticizing oil. Formulations given in patents reveal a fairly consistent range of compositions for SIS and HCR contents. Typically the HCR resin (taking the total midblock and end-block resin content) is within the range of about 50-70 wt.%. HCR supply has been an issue since about 2005 and may remain a challenge for years to come due to the switch to natural gas feedstock and slow, insufficient capacity increases in the market place. Adhesives formulators have expressed interest in both lower resin content formulations as well as moving from $C_5$ based isoprene to styrenic block copolymer (SBC) based on 1,3-butadiene monomer. Styrene-butadienestyrene (SBS) block copolymer can be used as a base-polymer for EAA however the lower $M_e$ (entanglement molecular weight) for polybutadiene and the propensity to cross-link under prolonged heat storage has made butadiene-based SBCs less attractive for this application. Furthermore, the industry has tried with varying success to use elastic polyolefins (usually with at least some fraction of hydrogenated SBC to add strength/elasticity) as a way to make a more sustainable formulation. However, the elastic polyolefin based EAA still require high HCR content and the performance is hampered by a strength mechanism depending on slow forming crystallinity.

There is industry concern about the sustainability of adhesives using $C_5$ monomers like isoprene. Reducing the amount of $C_5$ raw materials is highly desirable. Elastic attachment adhesives compositions use both $C_5$-based resins and $C_5$ monomers such as isoprene in the block copolymer styrene-isoprene-styrene and in the tackifier. The composition of the present invention shifts the raw material from $C_5$ usage to $C_4$ (butadiene) and $C_2$ (ethylene) and/or $C_3$ (propylene) for substitution of $C_5$ based resins, with the addition of amorphous polyolefin.

U.S. Patent 8,465,844 to Henkel AG discloses a hot melt adhesive comprising 15-35 wt.% of a radial S-I/B block copolymer, where S is styrene, I is isoprene, and B is butadiene; up to about 20 wt.% of a linear block copolymer such as SBS, SEBS, SEPS, and S-I/B-S and combinations thereof; and 30-70 wt.% tackifier. This composition has an average creep performance of about 4.1%. While this formula has excellent creep, the formulation uses SIS (based on $C_5$ monomer) as well as a large portion of $C_5$ resin. US2011/0244232A1 discloses continuous or continual in-line processes for forming adhesive polymer blend compositions as well as the application of those blends to a variety of substrates. Measures to improve creep are not disclosed. Thus there is a need to have a hot melt adhesive with good creep but uses substantially lower amounts of $C_5$ based raw materials.

**Summary of the Invention**

Elastic attachment adhesives used for laminating elastic fibers in diaper leg dam construction usually consist of high molecular weight SIS polymers and high resin content. The present invention formulations using lower resin content, amorphous polyolefins (APO) and low hydrogenated SBC content have now been developed. The combination of hydrogenated SBS also known as styrene-ethylene/butadiene-styrene (SEBS) and APO can offset resin content and still provide reasonable creep resistance and hot melt sprayability. The adhesive industry would view the shift from $C_5$ monomer dependence to $C_4/C_2$ as a more sustainable approach.

The present invention relates to a composition comprising:

(a) 10 to 20 wt.% of a block copolymer having the structure SEBS wherein S represents a polystyrene block, EB represents a hydrogenated polybutadiene block, wherein the block copolymer is selectively hydrogenated so that at least 90 percent of the conjugated diene double bonds have been reduced and 0-10 percent of the styrene double bonds have been reduced, wherein the block copolymer has a vinyl content of at least 60% before selective hydrogenation, wherein said SEBS has polystyrene end blocks with a peak molecular weight of 8 to 12 kg/mol, a peak molecular weight of 145-200 kg/mol, a polystyrene content of 15 to 30 wt.% and a melt flow rate of less than 12 grams per 10 min measured in accordance with ASTM D-1238 at 230°C under 2.16 kg mass, and wherein 10 wt.% or less of diblock polymer is present, wherein the peak molecular weight is measured with gel permeation chromatography (GPC) using polystyrene calibration standards as is done according to ASTM D5296;
(b) 40 to 50 wt.% of an amorphous polyolefin;
(c) 25 to 35 wt.% of a tackifier; and
(d) 3 to 8 wt.% of a maleated polypropylene oligomer or maleated SEBS
where the total composition is 100 wt.%.

The high vinyl content in the block copolymer used therein is at least 50%, preferably at least 60%, more preferably at least 65%, and most preferably more than 70% based on the total butadiene content.

Although the diblock copolymer is 10 wt.% or less, no diblock is most preferred. Even though no diblock is most preferred, when making a coupled high vinyl block copolymer, the range is generally between 1 to 10 wt.% diblock for the high vinyl block copolymer. An acurate range is 3 to 9 wt.% diblock and more accurate is 3 to 7 wt.%, and most accurate range is 5-7 wt.% diblock content in the high vinyl content block copolymer. Yet another embodiment of the present invention comprises a composition of 10 to 20 wt.% high peak molecular weight, high 1,2 butadiene-content SEBS having less than 10 wt.% diblock, 40 to 50 wt.% amorphous polyolefin, 25 to 35 wt.% tackifier, and 3 to 8 wt.% maleated polypropylene oligomer or maleated SEBS, where the total composition is 100 wt.%, wherein said SEBS has polystyrene end blocks with a peak molecular weight of 8 to 12 kg/mol, a polystyrene content of 15 to 30 wt.%, a vinyl content of at least 60%, a melt flow rate of less than 12 g/10 min. (230°C and 2.16 kg mass), according to ASTM D-1238, wherein said butadiene peak molecular weight is 128 to 148 kg/mol.

A further embodiment of the invention is an elastic laminate comprising the above mentioned composition. In such elastic laminate the composition is used as an elastic attachment adhesive to construct an elastic laminate. The elastic laminate comprises the elastic attachment adhesive, polar elastic fibers and non-polar thermoplastic films. The elastic laminates of the present invention have an initial creep of less than 25%.

## Description of the Preferred Embodiments

Any range cited herein includes the beginning number and the ending number and every number in between, as that is the very definition of a range.

As used in the specification and in the appended claims, the singular forms "a", "an", and "the" include the plural reference unless the context clearly indicates otherwise. For example, reference to "a conjugated diene" includes a plurality of such conjugated dienes.

The sequential preparation of block copolymers is well known. In a representative synthetic method, an initiator compound described later, is used to start polymerization with monoalkenyl arene monomer. The reaction is allowed to proceed until all of the monomer is consumed, resulting in a living homopolymer. To this living homopolymer is added a second monomer that is chemically different from the first, typically a conjugated diene. The living end of the first polymer serves as the site for continued polymerization, thereby incorporating the second monomer as a distinct second block in the linear polymer. In the present invention, the second block serves as the site for the continued polymerization upon addition of a different monomer, typically a monoalkenyl arene monomer. The copolymer so grown is living until terminated. Termination converts the living end of the copolymer into a non-propagating species, thereby rendering the polymer non-reactive toward monomer or coupling agent. Termination of polymerization can be accomplished by the addition of a small amount of alcohol. A polymer so terminated after polymerization of 2 different monomers is commonly referred to as a diblock copolymer. An unterminated, or living, monoalkenyl arene - conjugated diene diblock copolymer can be reacted with additional monoalkenyl arene monomer to form a linear sequential triblock copolymer. Triblock polymers so formed are referred to as ABA, where A represents a monoalkenyl arene block and B represents a conjugated diene block. Typical ABA block copolymers may be SBS, SIS, S-I/B-S, for example.

Starting materials for preparing the block copolymers of the present invention include the initial monomers. The monoalkenyl arene is styrene. Styrene is commercially available, and relatively inexpensive, from a variety of manufacturers.

The conjugated dienes for use herein are 1,3-butadiene and substituted butadienes such as isoprene, piperylene, 2,3-dimethyl-1,3-butadiene, and 1-phenyl-1,3-butadiene, or mixtures thereof. Of these, 1,3-butadiene is most preferred. As used herein, and in the claims, "butadiene" refers specifically to "1,3-butadiene".

The solvent used as the polymerization vehicle may be any hydrocarbon that does not react with the living anionic chain end of the forming polymer, is easily handled in commercial polymerization units, and offers the appropriate solubility characteristics for the product polymer. For example, non-polar aliphatic hydrocarbons, which are generally lacking in ionizable hydrogen make particularly suitable solvents. Frequently used are cyclic alkanes, such as cyclopentane, cyclohexane, cycloheptane, and cyclooctane, all of which are relatively non-polar. Other suitable solvents will be known to one skilled in the art and can be selected to perform effectively in a given set of process conditions, with temperature being one of the major factors taken into consideration.

Other important starting materials for anionic co-polymerizations include one or more polymerization initiators. In the present invention such initiators include, for example, alkyl lithium compounds and other organolithium compounds such as s-butyllithium, n-butyllithium, t-butyllithium, amyllithium and the like, including di-initiators such as the di-sec-butyl lithium an adduct of m-diisopropenyl benzene. Other such di-initiators are disclosed in U.S Pat. No. 6,492,469. Of the various polymerization initiators, s-butyllithium is preferred. The initiator can be used in the polymerization mixture (including monomers and solvent) in an amount calculated on the basis of one initiator molecule per desired polymer chain. The lithium initiator process is well known and is described in, for example, U.S. Pat. Nos. 4,039,593 and Re. 27,145.

An important aspect of the present invention is to control the microstructure or vinyl content of the conjugated diene in the selectively hydrogenated copolymer block B and in the softening modifier. The term "vinyl content" refers to the fact that a conjugated diene is polymerized via 1,2-addition (in the case of butadiene - it would be 3,4-addition in the case of isoprene). Although a pure "vinyl" group is formed only in the case of 1,2-addition polymerization of 1,3-butadiene, the effects of 3,4-addition polymerization of isoprene (and similar addition for other conjugated dienes) on the final properties of the block copolymer will be similar. The term "vinyl" refers to the presence of a pendant carbon-carbon double bond on the polymer chain as results from 1,2-addition of butadiene during polymerization. When referring to the use of butadiene as the conjugated diene, it is preferred that about 20 to about 85 mol percent of the condensed butadiene units in the copolymer block have 1,2-addition, or vinyl configuration as determined by proton NMR analysis. For selectively hydrogenated block copolymers, preferably about 30 to about 70 mol percent of the condensed butadiene units should have 1,2 configuration. This is effectively controlled by varying the relative amount of a microstructure modifying agent. Suitable microstructure agents and their ratios to lithium are disclosed and taught in U.S. Pat. Re 27,145.

The present invention has <10 wt.% diblock in the high molecular weight, high vinyl content, linear sequential S-EB-S block copolymer.

Although the amount of diblock copolymer is 10 wt. % or less in the high vinyl content block copolymer, no diblock is most preferred. For linear sequential S-EB-S block copoylmers it is possible to obtain a diblock content below 1 wt. %, and easily between 1-5 wt.%.

Hydrogenation generally for the conjugated diene can be carried out via any of the several hydrogenation or selective hydrogenation processes known in the prior art. In the case of S-B-S selective hydrogenation of the conjugated diene block converts this into S-EB-S. Hydrogenation of a butadiene block containing both 1,4-addition and 1,2-addition monomer units results in an ethylene/butylene structure and is referred to as EB. For example, such hydrogenation has been accomplished using methods such as those taught in, for example, U.S. Pat. Nos. 3,595,942; 3,634,549; 3,670,054; 3,700,633; and Re. 27,145. These methods operate to hydrogenate polymers containing aromatic or ethylenic unsaturation and are based upon operation of a suitable catalyst. Such catalyst, or catalyst precursor, preferably comprises a Group VIII metal such as nickel or cobalt which is combined with a suitable reducing agent such as an aluminum alkyl or hydride of a metal selected from Groups I-A, II-A and III-B of the Periodic Table of the Elements, particularly lithium, magnesium or aluminum. This preparation can be accomplished in a suitable solvent or diluent at a temperature from about 20°C to about 80°C. Other catalysts that are useful include titanium based catalyst systems.

Selective hydrogenation is according to the present invention carried out under such conditions that at least about 90 percent of the conjugated diene double bonds have been reduced, and between zero and 10 percent of the styrene double bonds have been reduced. Preferred ranges are at least about 95 percent of the conjugated diene double bonds reduced, and more preferably about 98 percent of the conjugated diene double bonds are reduced.

Once the hydrogenation is complete, it is preferable to extract the catalyst by stirring with the polymer solution a relatively large amount of aqueous acid (preferably 20 to 30 percent by weight), at a volume ratio of about 0.5 parts aqueous acid to 1 part polymer solution. Suitable acids include phosphoric acid, sulfuric acid and organic acids. This stirring is continued at about 50°C for about 30 to about 60 minutes while sparging with a mixture of oxygen in nitrogen. Care must be exercised in this step to avoid forming an explosive mixture of oxygen and hydrocarbons.

As used herein, the term "molecular weight(s)" refers to polystyrene equivalent, or apparent, molecular weight in g/mol of the polymer or block of the copolymer. The molecular weights referred to in this specification and claims can be measured with gel permeation chromatography (GPC) using polystyrene calibration standards, such as is done according to ASTM D5296. GPC is a well-known method wherein polymers are separated according to molecular size, the largest molecule eluting first. The chromatograph is calibrated using commercially available polystyrene molecular weight standards. The molecular weight of polymers measured using GPC so calibrated are styrene equivalent molecular weights, also referred to as apparent molecular weights. The styrene equivalent molecular weight may be converted to true molecular weight when the styrene content of the polymer and the composition and the vinyl content of the diene segments are known. The detector used is preferably a combination ultraviolet and refractive index detector. The molecular weights expressed herein are measured at the peak of the GPC trace and are commonly referred to as "peak molecular weights".

The high viscosity block copolymer of the present invention has the following characteristics: a SEBS having less than 10 wt.% diblock , having a PSC of 15 to 30 wt.%, preferably 18 to 23 wt.% and a styrene block molecular weight of 8 to 12 kg/mol., preferably 9 to 11 kg/mol and more preferably 9.2 to 10.2 kg/mol., and a vinyl content before selective hydrogenation of 60 to 75%, more preferably 65 to 75%, and most preferably 67 to 73%. The total peak "high" molecular weight of the block copolymer is 145 to 200 kg/mol., and more preferably 145 to 170 kg/mol., and most preferably 150 to 165 kg/mol. Additionally the block copolymer has a low viscosity as evidenced by a melt flow rate (at 230°C under 2.16 kg mass) of less than 12 grams per 10 min., preferably 1 to 10 grams per 10 min., and more preferably 1 to 6 grams per 10 min.

Tackifying resins include polystyrene block compatible resins and midblock compatible resins. The polystyrene block compatible resin may be selected from the group of coumarone-indene resin, polyindene resin, poly(methyl indene) resin, polystyrene resin, vinyltoluene-alphamethylstyrene resin, alphamethylstyrene resin and polyphenylene ether, in particular poly(2,6-dimethyl-1,4-phenylene ether). Such resins are e.g. sold under the trademarks "HERCURES", "ENDEX", "KRISTALEX", "NEVCHEM" and "PICCOTEX". Other suitable polystyrene compatible tackifiers are rosin esters, styrenated terpenes, polyterpenes, and terpene phenolics. These tackifiers are sold under the trademarks Sylvalite®, Sylvatac®, or Sylvamelt® for rosin esters, Zonatac® for styrenated terpenes, and Sylvares® for polyterpenes and terpene phenolics, all by Arizona Chemical Co.® Additionally, copolymers of various alkyl arene monomers such as alpha methyl styrene and para methyl styrene are a suitable tackifier. These tackifiers are sold under the name of ENDEX® by Eastman Chemical Co. One specific example of a polystyrene block compatible resin is Endex 160 which is an aromatic hydrocarbon resin having a ring and ball softening point of 159°C.

Resins compatible with the hydrogenated (mid) block may be selected from the group consisting of compatible $C_5$ hydrocarbon resins, hydrogenated $C_5$ hydrocarbon resins, styrenated $C_5$ resins, $C_5/C_9$ resins. Since the elimination or a significant reduction of $C_5$ tackifying resins is an important goal with the present invention, the following non-$C_5$ resins are preferred, namely: styrenated terpene resins, fully hydrogenated or partially hydrogenated $C_9$ hydrocarbon resins, rosins esters, rosins derivatives, dicyclopentadiene, and mixtures thereof. These resins are e.g. sold under the trademarks "WINGTAC", "REGALITE", "REGALREZ", "ESCOREZ", "ENDEX", "EASTOTAC", and "ARKON". Of specific utility in the present invention is Eastotac H-100W, manufactured by Eastman Chemical, which is a hydrogenated hydrocarbon resin having a ring and ball softening point of 100°C.

The amount of tackifying resin employed varies from about 5 to about 100 parts by weight per hundred parts by weight rubber, or block copolymer, preferably about 20 to about 50 parts by weight. Preferably the tackifier resins include both a polystyrene block compatible resin and a midblock compatible resin.

Tack is one of the most important properties for both pressure sensitive adhesives (PSA) and heat-seal adhesives. In spite of over 50 years of research on this topic, the concept of tack is not completely understood even though advancements have been made in its quantitative measurement. Tackifiers have an interesting effect on the viscoelastic properties of the base polymer. Adding compatible tackifiers can raise the glass transition, but lower the modulus above the glass transition of the blends. This is referred to as the Dahlquist criteria for tack. This is very different from the effect of the plasticizers; both materials are low molecular weight substances but tackifiers have a high Tg compared to the Tg of plasticizers which is typically lower than the base polymer Tg. Adding too much tackifier will cause tack to decrease sharply; this is not entirely surprising since tackifiers themselves are a brittle material at room temperature.

Amorphous polyolefins (APO) are polymers comprised of olefinic monomers and which have low to no crystallinity. They can be, for example, homopolymers of propylene, copolymers of propylene and ethylene, copolymers of propylene and 1-butene or higher alpha-olefins, or terpolymers of ethylene, propylene and butylene. The amount of crystallinity in the APO depends upon the monomers, their relative contents, and the polymerization catalyst. APOs typically melt over a broad range due to their broad distribution of crystallite sizes. Examples of commercial APOs are Eastoflex products from Eastman Chemical, Vestoplast products from Evonik Industries, and Rextac products from Rextac.

One issue with using amorphous polyolefins (APOs) is that there are a large number of possible candidates that could be applicable. However since one of the goals of the present invention is to eliminate or reduce the use of $C_5$-based tackifying resins, the preferred APO's are made from more plentiful, lower carbon number monomers such as ethylene and propylene. The preferred APO'S are ethylene, propylene and butylene homopolymers, and ethylene, propylene and butylene copolymers. For example ethylene/propylene copolymer, or ethylene/butene copolymer, or propylene/butene copolymers are suitable. Specifically, Rextac RT 2730 is a 1-butene copolymer APO having a ring and ball softening point of 107°C.

One other ingredient useful in the composition is a maleated polypropylene oligomer or maleated SEBS, or a mixture thereof. This maleated component is important because it affects adhesion to elastic fibers which are typically polar in nature, such as thermoplastic polyurethane. The amount employed is between 3 and 8 wt.% of the total weight of the composition. One function of the oligomer or maleated SEBS is to increase adhesion to the polar elastic fiber, this is a critical ingredient. A suitable maleated SEBS is FG1901 from Kraton Polymers having a bound maleic anhydride content of 1.4 to 2.0 wt.%. A suitable maleated polypropylene oligomer A-C 596 as described by Honeywell Performance Additives has 50 mg/KOH/g acid functionality and a drop point of 141°C. The amount of maleation in the polypropylene oligomer or maleated SEBS is at least 11 wt.% based on the total weight of this ingredient, and preferably between 11 and 15 wt.% maleation. Using A-C oligomers will provide reduced set times, as well as the additional benefits of increased adhesion and heat resistance.

Other ingredients such as mineral oils, fillers, and antioxidants may be included. Examples of useful oils are Puretol 35 a white mineral oil manufactured by Petro-Canada, and Drakeol 34 manufactured by Calumet Specialty Products. Examples of useful anti-oxidants are Ethanox 330, manufactured by Albermarle Corporation, and Irganox 1010 manufactured by BASF, both sterically hindered phenols.

The compositions of the present invention may be compounded further with other polymers, oils, fillers, reinforcements, antioxidants, stabilizers, fire retardants, antiblocking agents, lubricants and other rubber and plastic compounding ingredients without departing from the scope of this invention.

The composition of the present invention is particularly useful as an elastic attachment adhesive (EAA). As such, it is used to attach and bind elastic fibers into laminate constructions which can be used in personal hygiene articles. Elastic laminate constructions are typically made by sandwiching elastic fibers between thermoplastic sheets or films. The thermoplastic films can be polyolefin films. For example they can be polyethylene films, polypropylene films, and films made from olefin copolymers. The elastic fibers are typically polar in nature and can be composed of elastic polyurethanes and polyurethane-ureas such as found in Spandex fibers. It is important that any elastic attachment adhesive have good adhesion to both non-polar materials like polyolefin films and also polar materials like the elastic fibers. Such laminates are of particular application in diapers and adult incontinence articles. In these applications it is important that the laminate constructions do not experience excessive creep. In the context of this invention creep means the propensity for a stretched article to continue deforming after deformation to its initial stretched state. In other words it is the tendency of the elastic to separate from the material to which it is adhered. Theoretical zero percent creep means there is no separation of the elastic to the material to which it is adhered. For the maintenance of product integrity and performance lack of excessive creep is important. Otherwise, diapers and adult incontinence products may be subject to leaking. Elastic laminates of the present invention provide personal hygiene articles having an initial creep of less than 25%.

EXAMPLE

The ultimate test for EAAs is creep performance of the constructed laminate which is basically observing how far the attached elastic fibers (initially set under stress at 300% elongation) retract. Six elastic laminate constructs were cut to the approximate dimensions of 25mm x 400 mm. An elastic laminate constructs refers to a laminate that contains a top sheet of polyethylene film, 3 elastic fibers such as thermoplastic polyurethane fibers, and a polypropylene topsheet film. The elastic attachment adhesive bonds the elastic fibers together with the polyethylene and polypropylene films. The long side of this rectangular laminate is in the direction of the elastic fibers. Since the elastic laminate constructs are made while the fibers are stretched to 300% elongation, the appearance of the relaxed laminate is "puckered" with gathers.

One end of the cut elastic laminate construct is stapled to a cardboard testing board which is marked with three sets of lines. The first line is "zero" and is about 15mm from one edge of the cardboard. The second line is 285mm from the zero line and a third line at 300mm is also made. The elastic laminate construct is stapled between the zero mark and the edge of the cardboard. The construct is elongated to full extension (no gathers) to the 300mm line and marked with a pen. The construct is then allowed to contract until the mark sits over the 285mm line marking and then the opposite side is attached with a staple. This results in a fixed elastic laminate construct that is stretched to 95% of full extension. This is repeated until 6 elastic laminate constructs are like attached on the testing board. Two sets of cuts across the fibers are then made at the zero and 285mm marks so the elastic fibers are free to contract which is in effect the "creep" that is being measured. The cardboard containing the six constructs is put into an oven set at 37°C for a period of four hours. After removal from the oven, new marks are made measuring the distance the fibers have contracted when compared to the original marks, thus the % creep is reported as:

$$\textit{\% creep} = [\textit{Initial (@285mm mark)} - \textit{(Final/Initial)}] \ \textit{x 100; the average is reported}$$

For creep, lower numbers are better and looking into the patent literature reveals that about 25% or less creep is acceptable performance.

All the formulations in this report were prepared in a sigma blade mixer set to a temperature of about 180°C. Mixing times were generally in the range of 30 - 45 minutes. Since the APO fraction had poor solubility in aromatic solvents at room temperature, test samples were prepared by compression molding using a Carver press. The only formulations that were somewhat challenging to mix were the ones using Polymer A as the sole block copolymer - the mixing time was more in the range of one to 1.5 hours to effect complete mixing. Formula 1 and 2 are *comparative_examples* with a *coupled,* (SEB)$_n$X - type polymer, G1643, that has a *lower viscosity,* high vinyl content, PSC of 12 to 13 wt.% and a PS block peak molecular weight of 16.6 to 20.6 kg/mol., and a diblock content of between 5 and 7 wt.%, based on the total weight of G1643. The total peak mol. wt. is 144 kg/mol. G 1643 has a melt flow rate (230°C/2.16 kg) of 16 to 22 g/10 min. and a vinyl content of 75 to 81%. G1643 is not a suitable SEBS due to its lower MW that resulted in poor creep resistance.

Polymer A is a high vinyl, linear, sequential SEBS having *high viscosity,* a PSC of 20 wt.% and a PS block peak molecular weight of 9.7 kg/mol., a vinyl content of 70.5%, and a diblock content of less than 1 wt.%. The total peak molecular weight is 155 kg/mol. Polymer A has a melt flow rate (230°C/2.16 kg) of less than 1 gram per 10 min. The polybutadiene peak molecular weight is 136 kg/mol.

D1161 is a non-hydrogenated, linear, sequential styrene-isoprene-styrene block copolymer having a PSC of 13.5 to 16.5 wt.% and a total peak molecular weight of about 215 kg/mol.

A high melt flow rate indicates a low viscosity, and a low melt flow rate indicates a high viscosity. Thus, G1643 had a low viscosity compared to Polymer A.

| | Comp. F1 | Comp. F2 | F3 | F4 SIS Control |
|---|---|---|---|---|
| G1643 | 20 | 20 | | |
| Polymer A | | | 20 | |
| D1165 (control) SIS | | | | 25 |
| Rextac RT 2730 | 40 | 40 | 45 | |
| Endex 160 | | 5 | | 5 |
| Eastotac H-100W | 30 | 30 | 30 | 65 |
| AC596 | 10 | 5 | 5 | |
| Puretol 35 oil | | | | 5 |
| Viscosity at 149°C (cps) | 18,000 | 27,300 | 54,920 | 4,540 |
| Viscosity at 163°C (cps) | 9,300 | 14,800 | 18,980 | 2,390 |
| % Initial Creep @ 50 mg/lm/strand SW | 45.3% | 42.6% | 22.5% | 9.8% |

The above results show that Formula 3 has an acceptable creep, more comparable to SIS polymer compositions. Formulations 1 and 2 had unacceptably high creep at greater than 40%. Note that the control F4 employed 65 wt.% hydrogenated $C_5$ resin, whereas F3 employed only 30 wt.% of the $C_5$ resin. Thus, the inventive example, F3, exhibits a unique combination of low $C_5$ resin content and low creep.

Thus it is apparent that there has been provided, in accordance with the invention, a composition useful as an elastic attachment adhesive for the construction of elastic laminates having a creep less than 25%. These compositions contain SEBSamorphous polyolefin, tackifier, and maleated polyolefin, maleated SEBS or a mixture thereof, that fully satisfies the objects, aims, and advantages set forth above. While the invention has been described in conjunction with specific embodiments thereof, it is evident that many alternatives, modifications, and variations will be apparent to those skilled in the art in light of the foregoing description.

**Claims**

1. A composition comprising:

    a) 10 to 20 wt.% of a block copolymer having the structure SEBS wherein S represents a polystyrene block, EB represents a hydrogenated polybutadiene block, wherein the block copolymer is selectively hydrogenated so that at least 90 percent of the conjugated diene double bonds have been reduced and 0-10 percent of the styrene double bonds have been reduced, wherein the block copolymer has a vinyl content of at least 60% before selective hydrogenation, wherein said SEBS has polystyrene end blocks with a peak molecular weight of 8 to 12 kg/mol, a peak molecular weight of 145-200 kg/mol, a polystyrene content of 15 to 30 wt.% and a melt flow rate of less than 12 grams per 10 min measured in accordance with ASTM D-1238 at 230°C under 2.16 kg mass, and wherein 10 wt.% or less of diblock polymer is present, wherein the peak molecular weight is measured with gel permeation chromatography (GPC) using polystyrene calibration standards as is done according to ASTM D5296;
b) 40 to 50 wt.% of an amorphous polyolefin;
c) 25 to 35 wt.% of a tackifier; and
d) 3 to 8 wt.% of a maleated polypropylene oligomer or maleated SEBS

where the total composition is 100 wt.%.

2. The composition of claim 1, wherein the vinyl content is from 65 to 75%.

3. The composition of claim 1, wherein said amorphous alpha polyolefin is ethylene, propylene or butylene homopolymers, or ethylene, propylene or butylene copolymers, or mixtures thereof.

4. The composition of claim 3, wherein said ethylene, propylene and butylene copolymers may be ethylene/octene or propylene/octene or butylene/octene copolymers.

5. The composition of claim 1, wherein said tackifier resin is rosin esters, styrenated terpenes, polyterpenes, terpene phenolics or mixtures of two or more thereof.

6. The composition of claim 1, wherein said maleated polypropylene oligomer or maleated SEBS, or a mixture thereof possesses at least 1 wt.% maleation level.

7. The composition of claim 6, wherein the maleation level is from 1 to 15 wt.%.

8. An elastic laminate construction comprising the composition of any of claims 1 to 7.

9. An article comprising the laminate of claim 8.

10. The article of claim 9, which is a personal hygiene article selected from diapers or adult incontinence articles.


**Patentansprüche**

1. Zusammensetzung, umfassend:

a) 10 bis 20 Gew.-% eines Blockcopolymers mit der Struktur SEBS, wobei S einen Polystyrolblock darstellt, EB einen hydrierten Polybutadienblock darstellt, wobei das Blockcopolymer selektiv hydriert ist, so dass zumindest 90 Prozent der konjugierten Dien-Doppelbindungen reduziert wurden und 0-10 Prozent der Styrol-Doppelbindungen reduziert wurden, wobei das Blockcopolymer vor der selektiven Hydrierung einen Vinylgehalt von zumindest 60 % aufweist, wobei das SEBS Polystyrol-Endblöcke mit einem Peak-Molekulargewicht von 8 bis 12 kg/mol, einem Peak-Molekulargewicht von 145-200 kg/mol, einem Polystyrolgehalt von 15 bis 30 Gew.-% und einer Schmelzflussrate von weniger als 12 Gramm pro 10 min, gemessen nach ASTM D-1238 bei 230°C unter einer Masse von 2,16 kg aufweist und wobei 10 Gew.-% oder weniger Diblockpolymer vorhanden sind, wobei das Peak-Molekulargewicht mit Gelpermeationschromatographie (GPC) unter Verwendung von Polystyrol-Kalibrierungsstandards gemessen wird, wie nach ASTM D5296 durchgeführt;
b) 40 bis 50 Gew.-% eines amorphen Polyolefins;
c) 25 bis 35 Gew.-% eines Klebrigmachers; und
d) 3 bis 8 Gew.-% eines maleierten Polypropylen-Oligomers oder maleierten SEBS, wobei die Gesamtzusammensetzung 100 Gew.-% beträgt.

2. Zusammensetzung nach Anspruch 1, wobei der Vinylgehalt 65 bis 75 % beträgt.

3. Zusammensetzung nach Anspruch 1, wobei das amorphe alpha-Polyolefin Ethylen-, Propylen- oder Butylen-Homopolymere oder Ethylen-, Propylen- oder Butylen-Copolymere oder Gemische davon ist.

4. Zusammensetzung nach Anspruch 3, wobei die Ethylen-, Propylen- und Butylen-Copolymere Ethylen/Octen- oder Propylen/Octen- oder Butylen/Octen-Copolymere sein können.

5. Zusammensetzung nach Anspruch 1, wobei das klebrigmachende Harz Kolophoniumester, styrolisierte Terpene, Polyterpene, Terpenphenole oder Gemische von zwei oder mehr davon ist.

**6.** Zusammensetzung nach Anspruch 1, wobei das maleinierte Polypropylen-Oligomer oder maleierte SEBS oder ein Gemisch davon zumindest einen Maleinierungsgrad von zumindest 1 Gew.-% aufweist.

**7.** Zusammensetzung nach Anspruch 6, wobei der Maleinierungsgrad 1 bis 15 Gew.-% beträgt.

**8.** Elastische Laminatkonstruktion, umfassend die Zusammensetzung nach einem der Ansprüche 1 bis 7.

**9.** Artikel, umfassend das Laminat nach Anspruch 8.

**10.** Artikel nach Anspruch 9, der ein Hygieneartikel ausgewählt aus Windeln oder Inkontinenzartikeln für Erwachsene ist.

**Revendications**

**1.** Composition comprenant :

a) 10 à 20 % en poids d'un copolymère bloc ayant la structure SEBS dans laquelle S représente un bloc de polystyrène, EB représente un bloc de polybutadiène hydrogéné, dans laquelle le copolymère bloc est sélectivement hydrogéné de sorte qu'au moins 90 % des doubles liaisons conjuguées diéniques soient réduites et 0 à 10 % des doubles liaisons styréniques soient réduites, dans laquelle le copolymère bloc a une teneur en vinyle d'au moins 60 % avant hydrogénation sélective, dans laquelle ledit SEBS a des blocs terminaux de polystyrène avec un poids moléculaire maximal de 8 à 12 kg/mol, un poids moléculaire maximal de 145 à 200 kg/mol, une teneur en polystyrène de 15 à 30 % en poids et un indice de fluidité à chaud inférieur à 12 grammes par 10 min mesuré conformément à la norme ASTM D-1238 à 230°C sous 2,16 kg en masse, et dans laquelle 10 % en poids ou moins de polymère dibloc est présent, dans laquelle le poids moléculaire maximal est mesuré par chromatographie par perméation de gel (GPC) en utilisant des étalons de calibrage de polystyrène comme cela est fait conformément à la norme ASTM D5296 ;
b) 40 à 50 % en poids d'une polyoléfine amorphe ;
c) 25 à 35 % en poids d'un agent poisseux ; et
d) 3 à 8 % en poids d'un oligomère de polypropylène maléaté ou SEBS maléaté où la composition totale est de 100 % en poids.

**2.** Composition selon la revendication 1, dans laquelle la teneur en vinyle est de 65 à 75%.

**3.** Composition selon la revendication 1, dans laquelle ladite alpha-polyoléfine amorphe est un homopolymère d'éthylène, de propylène ou de butylène, ou un copolymère d'éthylène, de propylène ou de butylène, ou un mélange de ceux-ci.

**4.** Composition selon la revendication 3, dans laquelle lesdits copolymères d'éthylène, de propylène et de butylène peuvent être des copolymères d'éthylène/octène ou de propylène/octène ou de butylène/octène.

**5.** Composition selon la revendication 1, dans laquelle ladite résine collante est un ester de colophane, un terpène styréné, un polyterpène, un composé phénolique de terpène ou un mélange de deux ou plusieurs de ceux-ci.

**6.** Composition selon la revendication 1, dans laquelle ledit oligomère de polypropylène maléaté ou SEBS maléaté, ou un mélange de ceux-ci, possède au moins 1 % en poids de niveau de maléatation.

**7.** Composition selon la revendication 6, dans laquelle le niveau de maléatation est de 1 à 15% en poids.

**8.** Construction de stratifié élastique comprenant la composition selon l'une quelconque des revendications 1 à 7.

**9.** Article comprenant le stratifié selon la revendication 8.

**10.** Article selon la revendication 9, qui est un article d'hygiène personnelle choisi parmi les couches ou les articles pour l'incontinence adulte.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8465844 B, Henkel AG
- US 20110244232 A1
- US 6492469 B
- US 4039593 A
- US 27145 A

- US 3595942 A
- US 3634549 A
- US 3670054 A
- US 3700633 A